# EUROPÄISCHE PATENTSCHRIFT

(11) **EP 0 780 109 B1**
(45) Veröffentlichungstag und Bekanntmachung des Hinweises auf die Patenterteilung: **09.02.2000**
(21) Anmeldenummer: 96118031.2
(22) Anmeldetag: 11.11.1996
(51) Int. Cl.: A61F 13/58, A61F 13/62

(54) **Windelverschlussband**
Diaper closure tape
Bande de fermeture pour couche

(30) Priorität: 20.12.1995 DE 19547593
(43) Veröffentlichungstag der Anmeldung: 25.06.1997
(73) Patentinhaber: Koester GmbH & Co. KG, 96146 Altendorf (DE)
(72) Erfinder: Bräunig, Werner, 97514 Oberaurach (DE); Neugebauer, Robert, 91077 Neunkirchen am Brand (DE)
(74) Vertreter: Castell, Klaus, Dr.-Ing.

(56) Entgegenhaltungen:
- EP-A- 0 240 213
- EP-A- 0 444 353
- WO-A-92/01432
- WO-A-92/22274
- US-A- 4 410 325

## Beschreibung

Die Erfindung betrifft ein Windelverschlußband mit einem ersten Bandbereich, mit dem das Windelverschlußband an einem ersten Windelbereich befestigbar ist, und einem zweiten Bandbereich, mit dem das Windelverschlußband an einem zweiten Windelbereich befestigbar ist, wobei der erste Bandbereich eine Abdeckung aufweist, die einen Teil der Windelinnenseite des ersten Windelbereichs abdeckt.

Derartige Windelverschlußbänder sind weit verbreitet und werden zur Herstellung von Windeln für Erwachsene, die Inkontinenzprobleme haben, verwendet. Der Hauptanwendungsbereich derartiger Windelverschlußbänder liegt jedoch in der Herstellung von Wegwerfwindeln für Babys und Kleinkinder.

In der Praxis werden derartige Windelverschlußbänder mit einem ersten Bandbereich am hinteren Windelohr - dem ersten Windelbereich - unlösbar befestigt und ein zweiter Bandbereich, das sog. Fasteningtape, wird an einem zweiten Windelbereich, dem sog. vorderen Windelohr, lösbar befestigt. Hierzu wird das Fasteningtape vom übrigen am hinteren Windelohr befestigten Windelverschlußband abgezogen und an das vordere Windelohr angedrückt und somit festgeklebt.

Derartige Verschlußbänder sind beispielsweise aus der US-A-4,410,325 bekannt. Hierbei weist das Windelverschlußband einen ersten Bandbereich, mit dem das Windelverschlußband an einem ersten Windelbereich befestigbar ist, und einen zweiten Bandbereich, mit dem das Windelverschlußband an einem zweiten Windelbereich befestigbar ist, auf. Der erste Bandbereich ist dabei derart ausgebildet, daß er mit einer Abdeckung einen Teil der Windelinnenseite des ersten Windelbereichs abdeckt. Die Abdeckung weist auf ihrer Außenseite eine dehäsive Beschichtung auf. Vor Benutzung der Windel liegt der zweite Bandbereich mit einer Klebstoffschicht auf dieser dehäsiven Schicht auf und kann auf diese Weise ohne Weiteres vom übrigen am hinteren Windelohr befestigten Windelverschlußband abgezogen und an dem zweiten Windelbereich befestigt werden. Wird dieser zweite Bandbereich wieder von dem Windelohr bzw. dem zweiten Windelbereich abgezogen, so teilt sich die entsprechend eingestellte Klebstoffschicht und es verbleibt Klebstoff sowohl an dem zweiten Bandbereich als auch an dem vorderen Windelohr. Hierdurch soll eine sicheres Wiederverschließen gewährleistet werden.

Versuche haben jedoch gezeigt, daß bei der Verwendung derartiger Windelverschlußsysteme das vordere Windelohr den ersten und den zweiten Bandbereich nicht immer vollständig abdeckt. Dies wird besonders kritisch, wenn das Windelohr beim Anlegen der Windel zum Windelträger hin abknickt oder beim Tragen zusammengerafft wird und dadurch der Übergangsbereich zwischen erstem und zweitem Bandbereich auf der dem Windelträger zugewandten Seite des Windelverschlußbandes freigelegt wird. Dieser Übergangsbereich kann dann mit der Haut des Windelträgers in Berührung kommen und durch Druck oder Reibung Irritationen wie Rötungen verursachen.

Gerade die Verwendung von besonders dünnen Windeln mit wenig Vliesanteil und hohem Absorberanteil führt dazu, daß die Windelohren leicht abknicken und dann den Übergang nicht vollständig abdecken. Manche Windeln werden sogar mit einem rechteckigem Zellstoffkissen hergestellt. Dies führt dazu, daß die Windelohren keine Zellstoff-Füllung mehr aufweisen und somit besonders flexibel sind. Derartige rechteckige Zellstoffkissen führen zu hohen Einsparungen bei der Windelherstellung, haben jedoch den Nachteil, daß ein Abknicken der vorderen Windelohren beim Wickeln oder ein Zurückrollen der Windelohren während des Tragens besonders häufig auftritt.

Diese Problematik zeigt sich in besonderer Weise auch bei der US-A-4,410,325, bei welcher bei einem Abknicken oder Zusammenraffen sogar Klebstoff unmittelbar mit der Haut des Windelträgers in Berührung kommen kann.

Andererseits schlägt die EP-A1-0 240 213 eine Windelkonstruktion vor, bei welcher zwischen den beiden überlappenden Windelohrbereichen innere Befestigungsmittel vorgesehen sind, die ein Verschieben dieser überlappenden, durch das Windelverschlußband verbundenen Windelohren verhindern. Hierzu sollen die inneren Befestigungsmittel derart ausgelegt sein, daß sie ein Verschieben dieser beiden überlappenden Windelohrbereiche minimieren bzw. verhindern. Ein Abknicken oder ein Zurückrollen der Windelohren kann durch derartige Maßnahmen, die lediglich Scherkräften widerstehen, nicht verhindert werden.

Der Erfindung liegt die Aufgabe zugrunde, ein gattungsgem. Windelverschlußband so weiterzubilden, daß ein Kontakt zwischen den Übergangsbereichen auf der dem Windelträger zugewandten Seite des applizierten Windelverschlußbandes und den Körper des Windelträgers vermieden wird.

Diese Aufgabe wird dadurch gelöst, daß die von der Windelinnenseite abgewandte Seite der Abdeckung eine Klebezone aufweist.

Es hat sich gezeigt, daß die Verwendung einer Klebezone auf der dem Windelträger zugewandten Seite des applizierten Windelverschlußbandes dazu geeignet ist, das Windelohr so festzuhalten, daß es beim Tragen der Windel immer zwischen dem Übergangsbereich des Windelverschlußbandes und dem Körper des Windelträgers liegt. Durch die erfindungsgem. Klebezone wird ein Zurückschlagen des Windelohres auch dann vermieden, wenn das Windelohr besonders dünn ausgebildet ist, und somit wird gerade bei Windeln mit rechteckigem Zellstoffkissen jegliche Irritationsauslösung durch einen Kontakt zwischen Übergangsbereich und menschlicher Haut vermieden.

Vorteilhaft ist es, wenn die Klebezone etwa in der Mitte der Abdeckung angeordnet ist. Dies erlaubt eine Abdeckung eines Großteils des ersten Bandbereichs, der mit der Haut des Windelträgers in Berührung kommen könnte. In der Praxis wird das vordere Windelohr dann so an der Klebezone befestigt, daß zumindest ein Großteil der Abdeckung vorzugsweise sogar die gesamte Abdeckung durch das vordere Windelohr überdeckt wird. Dabei wird das hintere Windelohr so an das vordere Windelohr angeklebt, daß die Klebezone soweit im Endbereich des vorderen Windelohres liegt, daß ein Zurückschlagen des vorderen Windelohrs weitgehend vermieden wird.

Da die Windel nicht nur angelegt sondern nach ihrer Verwendung auch entfernt werden soll, ist es vorteilhaft, wenn die Klebezone wiederverschließbar gegen eine Windelaußenseite ist. Dadurch kann der erste Windelbereich ohne Beschädigung der Windelaußenhaut leicht wieder vom zweiten Windelbereich gelöst werden, um die Windel zu entfernen. Die Klebekraft zwischen Windelaußenhaut und Klebezone sollte zwischen 1 und 14 N/25mm liegen.

Vorzugsweise verbleibt die Klebezone nach einem ersten Kontakt mit dem zweiten Windelbereich beim Lösen der Klebeverbindung am zweiten Windelbereich.

Um eine gute Befestigung des ersten Bandbereichs am ersten Windelbereich zu gewährleisten, ist vorgeschlagen, daß der erste Bandbereich auch einen Teil der Windelaußenseite abdeckt. Diese Verbindung wird noch dadurch verstärkt, daß der erste Bandbereich eine Folie aufweist, die um einen Rand des ersten Windelbereichs herumführbar ist.

Das erfindungsgem. Windelverschlußband ist sowohl für mechanische, als auch für klebende Verschlußteile geeignet. Auch bei mechanischen Verschlußteilen treten häufig Irritationen beim Windelbenutzer im Übergangsbereich zwischen erstem und zweitem Bandbereich auf.

Wenn der erste oder zweite Bandbereich auf der Windelinnenseite einen mechanischen Verschlußteil aufweist und die Außenseite des ersten oder zweiten Bandbereichs so ausgestaltet ist, daß sie mit dem mechanischen Verschlußteil verbindbar ist, kann die benutzte Windel im eingerollten oder zusammengeklappten Zustand leicht gehalten werden, indem ein Windelverschlußband mit dem anderen Windelverschlußband mittels des mechanischen Verschlußteils verbunden wird. Besonders günstig ist es, wenn das Trägermaterial so ausgestaltet ist, daß es mit dem mechanischen Verschlußteil verbindbar ist.

Vorteilhaft ist es, wenn der zweite Bandbereich ein plastisches oder elastisches Material aufweist. Ein plastisches Material gibt bei einem starken Zug am Ende des zweiten Bandbereichs nach und reduziert somit den von der Windel auf den Körper des Windelträgers ausgeübten Druck. Ein elastisches Material tendiert sogar wieder in die ursprüngliche Form zurück und ist somit geeignet, flexibel im Bereich des Windelverschlußbandes Kräfte auszugleichen, die auf den Umfang der Windel ausgeübt werden.

Bei dem erfindungsgem. Windelverschlußband wird vorgeschlagen, daß der Teil des zweiten Bandbereiches plastisch oder elastisch ausgebildet ist, der beim Einklappen des zweiten Bandbereichs gegenüber der Klebezone liegt. Gerade dieser Teil des zweiten Bandbereichs sollte möglichst nicht mit Klebstoff beschichtet sein, da sonst zwei mit Klebstoff beschichtete Fläche aufeinander treffen.

Besonders vorteilhaft ist es, wenn der Teil des zweiten Bandbereiches, der beim Einklappen des zweiten Bandbereichs gegenüber der Klebezone liegt, eine Antihaft-Beschichtung aufweist. Eine Antihaft-Beschichtung in diesem Bereich erleichtert das Ablösen des eingeklappten zweiten Bandbereichs vom restlichen Windelverschlußband. Die Klebekraft der Klebezonen an der Antihaft-Beschichtung oder einer dehäsiven Ausrüstung sollte im Bereich zwischen 0,1 und 3,5 N/25mm liegen.

Als Antihaft-Beschichtung eignet sich einerseits eine zukaschierte mit Antihaftmitteln beschichtete Folie, welche bspw. durch Aufbringung einer Silikonschicht dehäsiv ausgerüstet ist. Andererseits ist es jedoch auch möglich, direkt auf das Trägermaterial des zweiten Bandbereichs des Windelverschlußbandes eine Antihaft-Beschichtung aufzubringen.

Eine Alternative zur Aufbringung eines Klebstoffs auf die Abdeckung liegt darin, daß die Klebezone durch Entfernen eines Teiles der Abdeckung beim Ausklappen des zweiten Bandbereiches freilegbar ist.

Außerdem wird ein Windelverschlußband vorgeschlagen, bei dem die Klebezone lösbar auf dem zweiten Bandbereich befestig ist und beim Aufklappen des zweiten Bandbereichs an der Abdeckung verbleibt.

Eine Antihaft-Ausrüstung zwischen Klebezone und Trägermaterial sorgt dafür, daß sich der Klebstoff vom Trägermaterial wieder abziehen läßt.

Ausführungsbeispiele des erfindungsgem. Windelverschlußbandes sind in der Zeichnung dargestellt und werden im folgenden näher beschrieben.

Es zeigt:
- Figur 1: eine Windel mit links einem herkömmlichen Windelverschlußband und rechts einem erfindungsgem. Windelverschlußband,
- Figur 2a: einen Schnitt durch ein herkömmliches Windelverschlußband,
- Figur 2b: ein am hinteren Windelohr befestigtes herkömmliches Windelverschlußband nach Figur 2c,
- Figur 2c: ein aufgeklapptes Windelverschlußband nach Figur 2b,
- Figur 2d: ein an einem hinteren und einem vorderen Windelohr befestigtes Windelverschlußband nach Figur 2c
- Figur 2e: ein am hinteren Windelohr leicht verschoben befestigtes herkömmliches Windelverschlußband nach Figur 2a,
- Figur 2f: ein aufgeklapptes Windelverschlußband nach Figur 2e,
- Figur 2g: ein an einem hinteren und einem vorderen Windelohr befestigtes Windelverschlußband nach Figur 2f,
- Figur 3a: ein am hinteren Windelohr befestigtes erfindungsgemäßes eingeklapptes Windelverschlußband,
- Figur 3b: ein am hinteren Windelohr befestigtes erfindungsgemäßes aufgeklapptes Windelverschlußband,
- Figur 3c: ein am hinteren Windelohr und einem vorderen Windelohr befestigtes erfindungsgemäßes Windelverschlußband,
- Figur 3d: das Verschlußband nach Figur 3c mit eingeklapptem vorderem Windelohr,
- Figur 4a: ein herkömmliches Windelverschlußband,
- Figur 4b: ein aufgeklapptes herkömmliches Windelverschlußband,
- Figur 5a: ein erfindungsgem. Windelverschlußband mit erfindungsgemäßer Klebezone,
- Figur 5b: ein aufgeklapptes Windelverschlußband mit erfindungsgemäßer Klebezone,
- Figur 6a: ein alternatives erfindungsgem. Windelverschlußband
- Figur 6b: ein aufgeklapptes alternatives Windelverschlußband nach Figur 6a,
- Figur 7a: ein erfindungsgem. Windelverschlußband mit mechanischem Verschluß,
- Figur 7b: ein aufgeklapptes Windelverschlußband nach Figur 7a,
- Figur 8: ein erfindungsgem. Windelverschlußband mit mechanischem Verschluß und vom Trägermaterial ablösbarem Klebebereich.

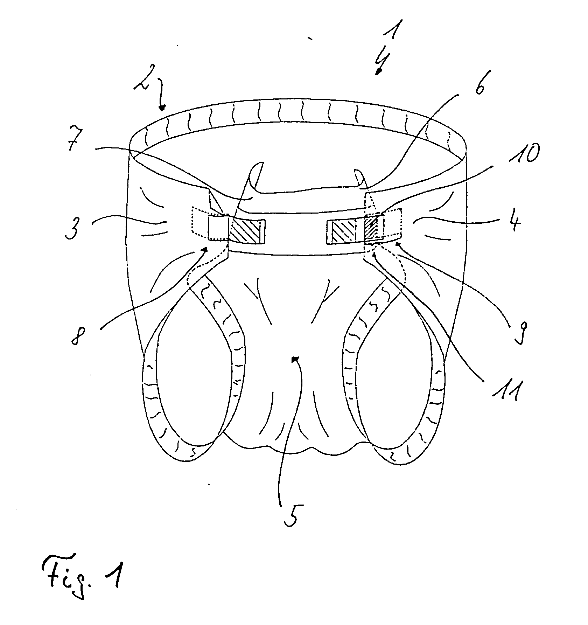

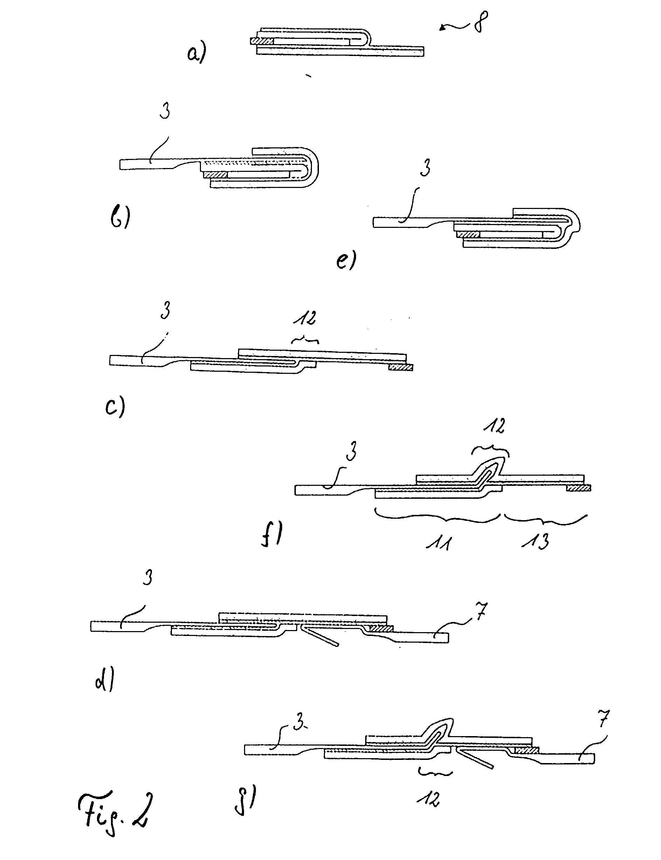

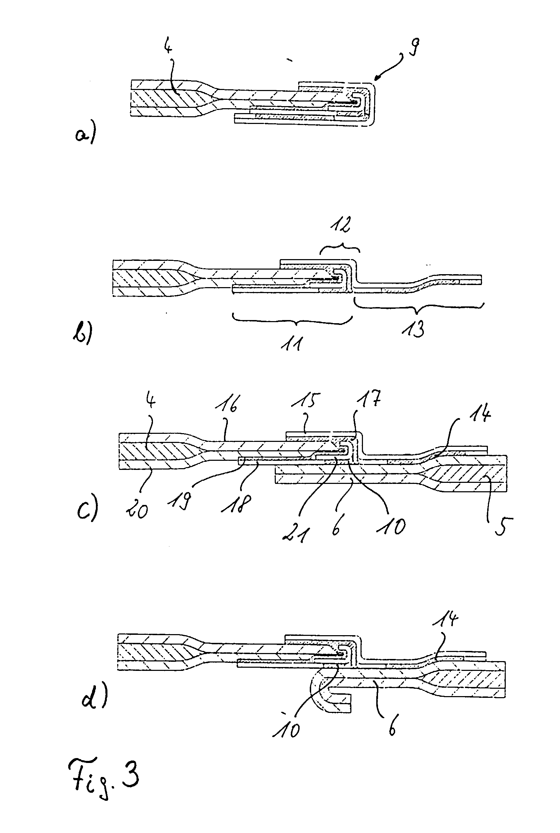

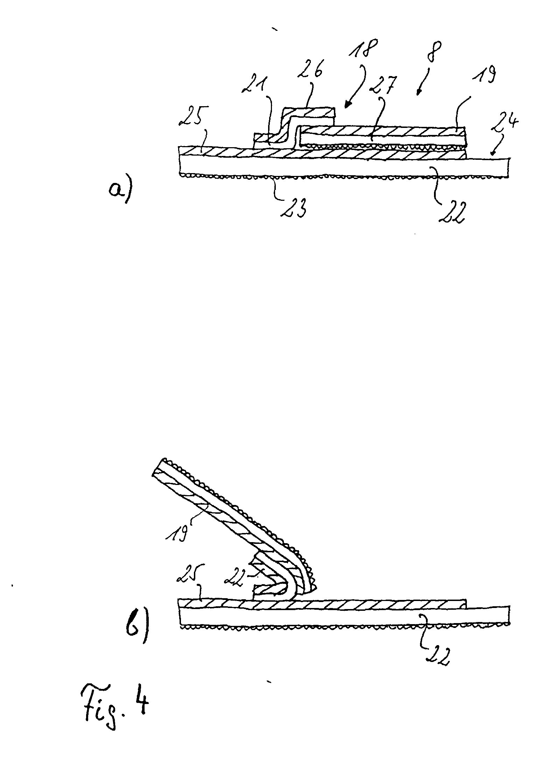

Die in Figur 1 gezeigte Windel 1 hat einen ersten Windelbereich 2 mit zwei hinteren Windelohren 3 und 4 und einen zweiten Windelbereich 5 mit zwei vorderen Windelohren 6 und 7. Die hinteren Windelohren 3 und 4 des ersten Windelbereichs 2 sind mittels Windelverschlußbändern 8 und 9 an den vorderen Windelohren 6 und 7 des zweiten Windelbereichs 5 befestigt.

Das linke Windelverschlußband 8 ist ein konventionelles Windelverschlußband, das zwar dazu geeignet ist, das hintere Windelohr 3 am zweiten Windelbereich 5 zu befestigen, bei dem jedoch leicht das vordere Windelohr 7 soweit umknickt, daß der zum Teil harte, dicke, irritationsauslösende Übergangsbereich 12 in Berührung mit der Haut des Windelträgers kommen kann. Gerade bei besonders dünn gefertigten Windelohren 7 ist das beschriebene Umschlagen des Windelohrs sehr häufig und der Kontakt zwischen Windelverschlußband 8 und der Haut des Windelträgers führt zu Irritationen, wie bspw. Rötungen.

Das auf der rechten Seite abgebildete Windelverschlußband 9 hat eine Klebezone 10 an einem ersten Bandbereich 11, mit dem das Windelverschlußband 9 am hinteren Windelohr 4 befestigt ist. Diese Klebezone 10 ist so angeordnet, daß sie beim Anlegen der Windel auf dem ersten Bandbereich 11 zwischen dem vorderen Windelohr 6 und dem hinteren Windelohr 4 liegt. Dadurch wird ein weiterer Teil des vorderen Windelohrs 6 am hinteren Windelohr 4 befestigt, um ein Umschlagen des vorderen Windelohrs 6 zu verhindern oder zumindest soweit zu begrenzen, daß der harte Übergangsbereich 12 (vgl. Fig. 2) des Windelverschlußbandes 9 während der gesamten Tragedauer abgedeckt ist und dadurch ein direkter Kontakt des Übergangsbereiches 12 mit der Haut des Windelträgers ausgeschlossen werden kann.

Figur 2 zeigt, wie im Gegensatz zu einem in den Figuren b - d gezeigten ordnungsgemäß befestigten Windelverschlußband schon ein kleine Ungenauigkeit beim Applizieren des Windelverschlußbandes an das hintere Windelohr zu den in den Figuren e, f, g gezeigten Unrelgelmäßigkeiten führen kann. Dadurch entstehen schon durch kleinste Befestigungsfehler Unebenheiten, die in einem Übergangsbereich 12 zwischen erstem Bandbereich 11 und zweiten Bandbereich 13 zu besonderen Verhärtungen des Windelverschlußbandes führen.

Figur 3a zeigt einen Schnitt durch das am hinteren Windelohr 4 befestigte Windelverschlußband 9. Das Windelverschlußband 9 ist um das Ende des hinteren Windelohres 4 herumgelegt und wird in dieser Form nach Fertigstellung der Windel zum Endverbraucher transportiert.

Der Endverbraucher, zieht, wie in Figur 3b gezeigt, einen zweiten Bandbereich 13 vom ersten Bandbereich 11 ab, um ihn, wie in Figur 3c gezeigt, am zweiten Windelbereich 5 oder am vorderen Windelohr 6 des zweiten Windelbereichs 5 zu befestigen. Dazu weist der zweite Bandbereich 13 eine Klebstoffbeschichtung 14 auf, mittels der der zweite Bandbereich 13 lösbar am zweiten Windelbereich 5 befestigbar ist.

Der erste Bandbereich 11 besteht aus einem Teil 15, das an der Außenseite 16 des hinteren Windelohres 4 mittels einer Klebstoffbeschichtung 17 befestigt ist und einer Abdeckung 18, die mittels einer Klebstoffbeschichtung 19 an der Innenseite 20 des hinteren Windelohres 4 befestigt ist. Die Abdeckung 18 des ersten Bandbereiches 11 weist eine Folie 21 auf, die um das Ende 12 des hinteren Windelohres 4 herumgeführt ist. Die Folie 21, die die Abdeckung 18 mit dem äußeren Bandbereich verbindet und einen Teil der Abdeckdung 18 bildet, ist auf der vom hinteren Windelohr 4 abgewandten Seite mit Klebstoff beschichtet und bildet dadurch eine Klebezone 10, auf der von der Windelinnenseite 20 abgewandten Seite der Abdeckung 18.

Diese Klebezone 10 ist, wie in Figur 3d gezeigt, dazu geeignet, ein Umschlagen des vorderen Windelohres 6 soweit zu begrenzen, daß ein Kontakt zwischen dem Übergangsbereich 12 und der Haut des Windelträgers vermieden wird.

Figur 4a zeigt im Gegensatz zur Figur 3 schematisch ein herkömmliches Windelverschlußband 8. Dieses Windelverschlußband 8 besteht aus einem Fasteningträgermaterial 22, das auf der Unterseite eine dehäsive Ausrüstung 23, wie bspw. eine Silikonschicht aufweist. Auf der darüberliegenden Seite ist das Fasteningträgermaterial 22 bis auf eine Anfaßzone 24 mit Klebstoff 25 beschichtet. An diese Klebstoffbeschichtung 25 ist eine Folie 21 angeklebt, die auf ihrer Oberseite wiederum mit Klebstoff 22 beschichtet ist. Die Folie 21 bildet mit einer weiteren, auf ihrer Oberseite klebstoffbeschichteten Folie oder einem Papier 27 die Abdeckung 18, die - wie in Figur 4b gezeigt - aufgeklappt werden kann, um zwischen der Klebstoffbeschichtung 19 der Folie 27, der Klebstoffbeschichtung 26 der Folie 21 und der Klebstoffbeschichtung 25 des Fasteningträgermaterials 22 ein hinteres Windelohr 4 festzukleben. Das vordere Windelohr 7 kann dann an der freigelegten Klebstoffbeschichtung 25 des Fasteningträgermaterials 22 befestigt werden.

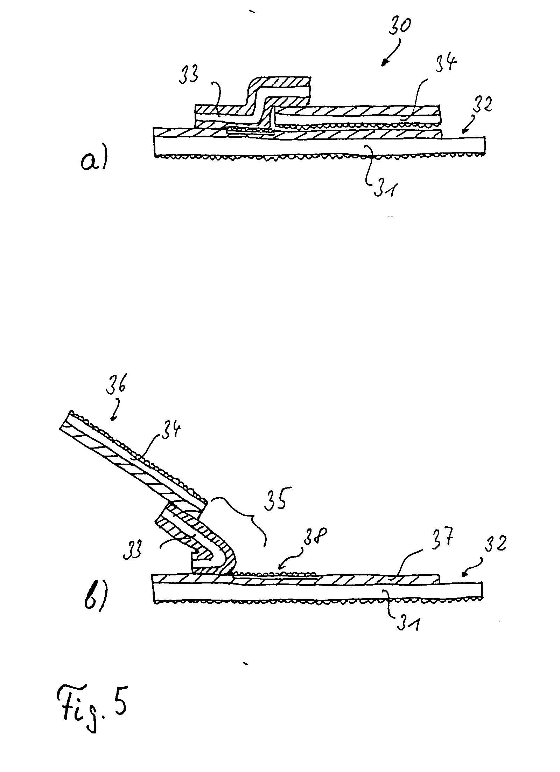

Figur 5 zeigt ein im wesentlichen dem in Figur 3 gezeigten Windelverschlußband 9 entsprechendes Windelverschlußband 30. Dieses Windelverschlußband 30 ist in Figur 5a als Schnitt durch die Rollenware und in Figur 5b als aufgeklapptes Windelverschlußband 30 kurz vor der Befestigung am hinteren Windelohr 4 dargestellt. Das Fasteningträgermaterial 31 ist wiederum bis auf einen Anfaßbereich 32 mit Klebstoffbeschichtet und eine Folie 33 hält, wie auch in den vorhergehenden Ausführungsbeispielen, eine weitere Folie 34.

Wenn die Folie 34 jedoch, wie in Figur 5b gezeigt, von dem Fasteningträgermaterial 31 abgezogen wird, löst sich auch zum Teil die Folie 33 vom Fasteningträgermaterial 31, so daß eine Klebezone 35 an der weggeklappten Abdeckung 36 freigelegt wird. Diese Klebezone 35, dient, wie oben beschrieben, zur Befestigung des vorderen Windelohres. Um ein begrenztes Lösen der Folie 33 von dem Fasteningträgermaterial 31 zu bewirken, ist die Klebstoffbeschichtung 37 auf dem Teil 38 des Fasteningträgermaterials 31, der beim Einklappen des zweiten Bandbereichs gegenüber der Klebezone 35 liegt, dehäsiv ausgerüstet, d. h. bspw. mit einer Silikonschicht überdeckt oder mit einer einseitig silikonisierten Folie abgedeckt, wobei die silikonisierte Seite zur Klebezone 35 gewandt ist.

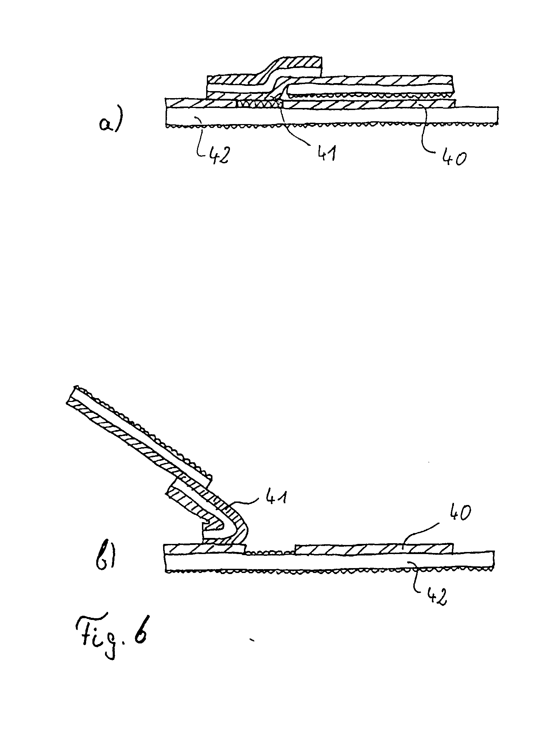

Figur 6 zeigt ein weiteres Ausführungsbeispiel, das im wesentlichen dem Ausführungsbeispiel nach Figur 5 entspricht und bei dem die Klebstoffbeschichtung 40 im Bereich gegenüber die Klebezone 41 unterbrochen ist und zwischen den beiden Teilen der Klebebeschichtung 40 gegenüber der Klebezone 41 das Fasteningträgermaterial 42 antihaftbeschichtet ist.

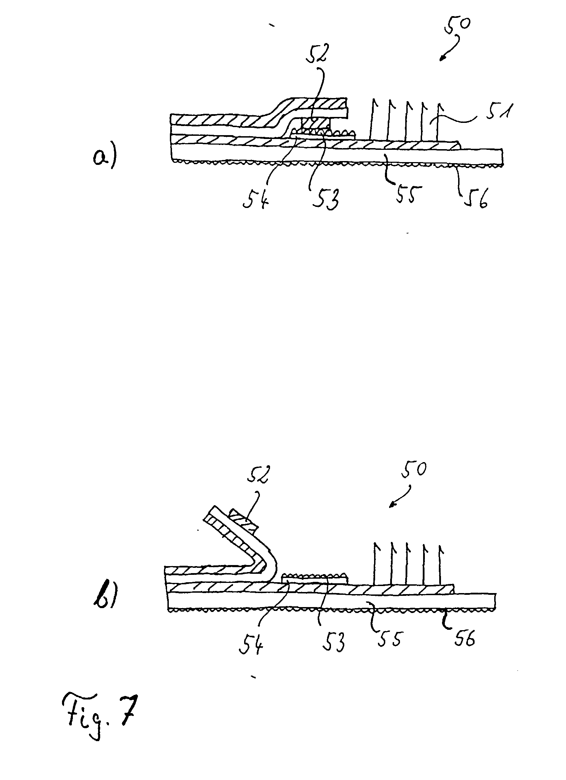

Figur 7 zeigt ein weiteres Ausführungsbeispiel eines Windelverschlußbandes 50, bei dem sowohl ein mechanischer Verschlußteil 51 als auch eine erfindungsgemäße Klebezone 52 vorgesehen sind. Figur 7a zeigt das erfindungsgem. Windelverschlußband 50 als Schnitt durch die Rollenware, bei der die Klebezone 52 auf einer antihaftbeschichteten Folie 54 oder einem antihaftbeschichtem Papier 53 aufliegt und Figur 7b zeigt ein aufgeklapptes Windelverschlußband 50, bei dem die Klebezone 52 freigelegt ist. Hierbei ist das Trägermaterial 55 oder zumindest seine äußere Oberfläche 56 aus einem Material hergestellt, das mit dem mechanischen Verschlußteil 51 zusammenwirken kann. Als Trägermaterial oder Beschichtung sind u.a. textile, samtige oder schlingenartige Materialien wie Gewebe oder Gewirke geeignet.

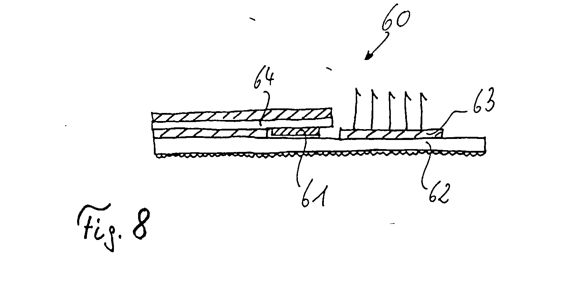

Figur 8 zeigt ein weiteres Beispiel zur Anbringung der Klebezone 61 am Windelverschlußband 60. Ein Teil der auf dem Fasteningträgermaterial 62 aufgebrachten Klebstoffbeschichtung 63, das zwischen dem Fasteningträgermaterial 62 und der Abdeckung 64 liegt, löst sich beim Abziehen der Abdeckung 64 vom Fasteningträgermaterial 62 und dient nach Anbringung des Windelverschlußbandes 60 am hinteren Windelohr als Klebezone 61. Das Ablösen der Klebstoffbeschichtung 61 vom Fasteningträgermaterial 62 kann bspw. durch eine partielle dehäsive Ausrüstung zwischen der Klebezone 61 und dem Fasteningträgermaterial 62 forciert werden.

## Patentansprüche

1. Windelverschlußband (9) mit einem ersten Bandbereich (11) mit dem das Windelverschlußband (9) an einem ersten Windelbereich (2) befestigbar ist, und einem zweiten Bandbereich (13), mit dem das Windelverschlußband (9) an einem zweiten Windelbereich (5) befestigbar ist, wobei der erste Bandbereich (11) eine Abdeckung (18) aufweist, die einen Teil der Windelinnenseite (20) des ersten Windelbereiches (2) abdeckt, **dadurch gekennzeichnet, daß** die von der Windelinnenseite (20) abgewandte Seite der Abdeckung (18) eine Klebezone (10) aufweist.

2. Windelverschlußband nach Anspruch 1, **dadurch gekennzeichnet, daß** die Klebezone (10) wiederverschließbar gegen eine Windelaußenseite ist.

3. Windelverschlußband nach Anspruch 1 oder 2, **dadurch gekennzeichnet, daß** die Klebezone (10) nach einem ersten Kontakt mit dem Windelbereich (5) beim Lösen der Klebeverbindung am zweiten Windelbereich (5) verbleibt.

4. Windelverschlußband nach einem der vorhergehenden Ansprüche, **dadurch gekennzeichnet, daß** der zweite Bandbereich (13) einen klebenden Verschlußteil (14) aufweist.

5. Windelverschlußband nach einem der vorhergehenden Ansprüche, **dadurch gekennzeichnet, daß** der erste oder zweite Bandbereich (13) auf der Windelinnenseite einen mechanischen Verschlußteil aufweist und die Außenseite des ersten oder zweiten Bandbereichs so ausgebildet ist, daß sie mit dem mechanischen Verschlußteil verbindbar ist.

6. Windelverschlußband nach einem der vorhergehenden Ansprüche, **dadurch gekennzeichnet, daß** der zweite Bandbereich ein plastisches oder elastisches Material aufweist.

7. Windelverschlußband nach einem der vorhergehenden Ansprüche, **dadurch gekennzeichnet, daß** der Teil des zweiten Bandbereichs, der beim Einklappen des zweiten Bandbereichs gegenüber der Klebezone liegt, eine Antihaft-Beschichtung aufweist.

8. Windelverschlußband nach Anspruch 7, **dadurch gekennzeichnet, daß** die Antihaftbeschichtung direkt auf einem Trägermaterial des Verschlußbandes oder auf einer auf das Trägermaterial aufklebbaren Folie aufgebracht ist.

9. Windelverschlußband nach einem der vorhergehenden Ansprüche, **dadurch gekennzeichnet, daß** die Klebezone lösbar auf dem zweiten Bandbereich (13) befestigt ist und beim Aufklappen des zweiten Bandbereichs (13) an der Abdeckung (18) verbleibt.

10. Windelverschlußband nach einem der vorhergehenden Ansprüche **dadurch gekennzeichnet, daß** das Verschlußband ein Trägermaterial aus thermoverfestigten Polypropylenvlies aufweist.

## Claims

1. Diaper closure tape (9) having a first tape area (11) to which the diaper closure tape (9) can be attached on a first diaper area (2), and a second tape area (13) to which the diaper closure tape (9) can be attached, whereby the first tape area (11) comprises a covering (18) which covers a part of the diaper inner side (20), characterised in that the side of the covering (18) averted from the diaper inner side (20) comprises an adhesion zone (10).

2. A diaper closure tape as claimed in claim 1, characterised in that the adhesion zone (10) can be reclosed against a diaper outside.

3. A diaper closure tape as claimed in claim 1 or 2, characterised in that the adhesion zone (10) remains on the second diaper area (5) after initial contact with the diaper area (5) when the adhesive connection is released.

4. A diaper closure tape as claimed in any one of the foregoing claims, characterised in that the second tape area (13) comprises a viscous closure piece (14).

5. A diaper closure tape as claimed in any one of the foregoing claims, characterised in that the first or second tape area (13) comprises a mechanical closure piece on the diaper inner side, and in that the outside of the first or second tape area is designed such that it can be connected to the mechanical closure piece.

6. A diaper closure tape as claimed in any one of the foregoing claims, characterised in that the second tape area comprises a plastic or elastic material.

7. A diaper closure tape as claimed in any one of the foregoing claims, characterised in that the part of the second tape area which lies opposite the adhesion zone when the second tape area is folded comprises an anti-stick coating.

8. A diaper closure tape as claimed in claim 7, characterised in that the anti-stick coating is applied directly to a carrier material of the closure tape, or to a sheet which can be stuck to the carrier material.

9. A diaper closure tape as claimed in any one of the foregoing claims, characterised in that the adhesion zone is attached detachably to the second tape area (13) and remains on the covering (18) when the second tape area (13) is unfolded.

10. A diaper closure tape as claimed in any one of the foregoing claims, characterised in that the closure tape comprises a carrier material made of thermobonded polypropylene fabric.

## Revendications

1. Bande de fermeture (9) pour couche, comprenant une première zone de bande (11) permettant la fixation de la bande de fermeture (9) pour couche à une première partie de couche (2), et une deuxième zone de bande (13) permettant la fixation de la bande de fermeture (9) pour couche à une seconde partie (5) de la couche, la première zone (11) de la bande présentant un élément de recouvrement (18) qui recouvre une partie de la face intérieure (20) de la première partie (2) de la couche, caractérisée en ce que la face de l'élément de recouvrement (18) orientée à l'opposé de la face intérieure (20) de la couche présente une zone adhésive (10) .

2. Bande de fermeture pour couche selon la revendication 1, caractérisée en ce que la zone adhésive (10) peut être réappliquée contre une face extérieure de la couche.

3. Bande de fermeture pour couche selon la revendication 1 ou 2, caractérisée en ce qu'après un premier contact avec la partie de couche (5), la zone adhésive (10) subsiste lorsque la liaison adhésive est supprimée sur la seconde partie de couche (5).

4. Bande de fermeture pour couche selon l'une des revendications qui précèdent, caractérisée en ce que la seconde zone de bande (13) présente un élément de fermeture adhésif (14).

5. Bande de fermeture pour couche selon l'une des revendications qui précèdent, caractérisée en ce que la première ou la seconde zone de bande (13) présente un élément de fermeture mécanique sur la face intérieure de la couche, et en ce que la face extérieure de la première ou de la seconde zone de bande est conçue pour pouvoir être raccordée à l'élément de fermeture mécanique.

6. Bande de fermeture pour couche selon l'une des revendications qui précèdent, caractérisée en ce que la seconde zone de bande présente une matière plastique ou élastique.

7. Bande de fermeture pour couche selon l'une des revendications qui précèdent, caractérisée en ce que la partie de la seconde zone de bande positionnée en regard de la seconde zone adhésive lors du rabattement de la seconde zone de bande présente un revêtement anti-adhésif.

8. Bande de fermeture pour couche selon la revendication 7, caractérisée en ce que le revêtement anti-adhésif est appliqué directement sur une matière de support de la bande de fermeture ou sur une feuille qui peut être collée à la matière de support.

9. Bande de fermeture pour couche selon l'une des revendications qui précèdent, caractérisée en ce que la zone adhésive est fixée à la seconde zone de couche (13) de manière à pouvoir être détachée et en ce qu'elle reste sur l'élément de recouvrement (18) lors du rabattement de la seconde zone de bande (13).

10. Bande de fermeture pour couche selon l'une des revendications qui précèdent, caractérisée en ce que la bande de fermeture présente une matière de support à base d'un non tissé de polypropylène solidifié à la chaleur.
